# EUROPEAN PATENT SPECIFICATION

(11) **EP 2 445 486 B1**
(45) Date of publication and mention of the grant of the patent: **08.11.2017**
(21) Application number: 10725949.1
(22) Date of filing: 21.06.2010
(51) Int. Cl.: A61K 9/20, A61K 9/28, A61K 31/192

(54) **SODIUM IBUPROFEN TABLETS AND METHODS OF MANUFACTURING PHARMACEUTICAL COMPOSITIONS INCLUDING SODIUM IBUPROFEN**
NATRIUMIBUPROFENTABLETTEN UND VERFAHREN ZUR HERSTELLUNG PHARMAZEUTISCHER ZUSAMMENSETZUNGEN MIT NATRIUMIBUPROFEN
COMPRIMÉS D'IBUPROFÈNE SODIQUE ET PROCÉDÉS DE FABRICATION DE COMPOSITIONS PHARMACEUTIQUES COMPRENANT DE L'IBUPROFÈNE SODIQUE

(30) Priority: 22.06.2009 US 219149 P
(43) Date of publication of application: 02.05.2012
(73) Proprietor: Wyeth LLC, New York, NY 10017-5755 (US)
(72) Inventor: SEYER, Jeffery, J., Richmond VA 23236 (US); CONDER, Amy, L., Midlothian VA 23112 (US); TAYLOR, Angela, P., Clayton NC 27527 (US); SHAW, Bonny, Rene, New Kent VA 23124 (US)
(74) Representative: Pfizer
(86) International application number: PCT/US2010/039346
(87) International publication number: WO 2011/005478

(56) References cited:
- WO-A1-92/20334
- WO-A1-2004/035024
- WO-A1-2009/101258
- WO-A2-97/30699

## Description

### FIELD OF THE INVENTION

The present invention relates to novel sodium ibuprofen cores and coated tablet/caplet compositions having a low sodium content relative to other commercially available sodium ibuprofen dosage forms and methods of manufacturing such sodium ibuprofen cores and corresponding pharmaceutically acceptable compositions. The sodium ibuprofen cores and coated core sodium ibuprofen compositions and formulation are advantageous because it allows for the formation of tablet/caplet cores having a maximum daily sodium content for a patient of less than 140 mg/day, based on the tablet/caplet compositions and further provides sodium ibuprofen tablet/ caplet cores and corresponding coated sodium ibuprofen cores exhibiting improved physical stability, high tablet/caplet hardness and high sodium ibuprofen core strength, coupled and balanced with excellent dissolution and bioavailability characteristics. The pharmaceutically acceptable sodium ibuprofen core and coated core compositions, formulations and processes of manufacturing thereof are further advantageous because they can be commercially manufactured in large quantities without an unacceptable number of defective tablets.

### BACKGROUND OF THE INVENTION

Solid dosage forms of ibuprofen are well known. Although tablet compositions of ibuprofen are commercially available, poor tablet compression, stability and disintegration remain critical formulation issues. While it is generally the case that tablets formed by compression under low compression force also dissolve more rapidly than tablets formed by high compression force, tablets produced under lower pressure often have a high degree of friability. International Patent Publication No. WO 2004/035024 A1 is a typical example of a dosage form of sodium ibuprofen. However, the tablets only possess sufficient, not optimal hardness and contain large total sodium content, which is not advantageous to patients, especially frequent and daily users of such over the counter medicaments. Further, crumbling and breakage of such tablets prior to ingestion may lead to uncertainty as to the dosage of active ingredient per tablet and core defects, including picking and sticking. Furthermore, high friability also causes tablet breakage leading to waste during factory handling.

The present invention addresses these and other problems associated with the prior art. The invention provides an improved sodium ibuprofen tablet core having low sodium content relative to commercially available sodium ibuprofen dosage forms and further provides tablets/caplets having optimal hardness balanced with excellent dissolution, low friability and high stability and which have the added advantage of cost-effective methods of manufacture.

### 1. SUMMARY OF THE INVENTION

The present invention advantageously provides a pharmaceutical composition comprising a core containing sodium ibuprofen having low sodium content, based on the composition. The invention provides the pharmaceutical composition in the form of a tablet or caplet further comprising at least one coating, the core comprises at least two binders including microcrystalline cellulose and mannitol; and the sodium ibuprofen of the core is present in the form of a dihydrate. The invention provides the pharmaceutical composition, having a total daily sodium content for a patient of less than 140 mg/day, including about 134 mg/day or less and provides a sodium content of less than 23 mg/dosage unit available daily in six dosages to a patient in need of treatment with sodium ibuprofen. Pharmaceutically acceptable compositions and corresponding coated tablets and caplets are manufactured having high sodium ibuprofen core strength and hardness, having low sodium content relative to commercially available sodium ibuprofen formulations and further provide sodium ibuprofen tablets that have excellent dissolution profiles and bioactivity.

The accompanying Detailed Description, Examples and Drawings further elaborates the present invention and its advantages.

### DESCRIPTION OF THE DRAWINGS

TABLE 1 shows a representative composition of a sodium ibuprofen tablet drug product and the function of the excipients in the formulation.
TABLE 2 shows a representative composition of a sodium ibuprofen tablet drug product containing lactose and the function of the excipients in the formulation.

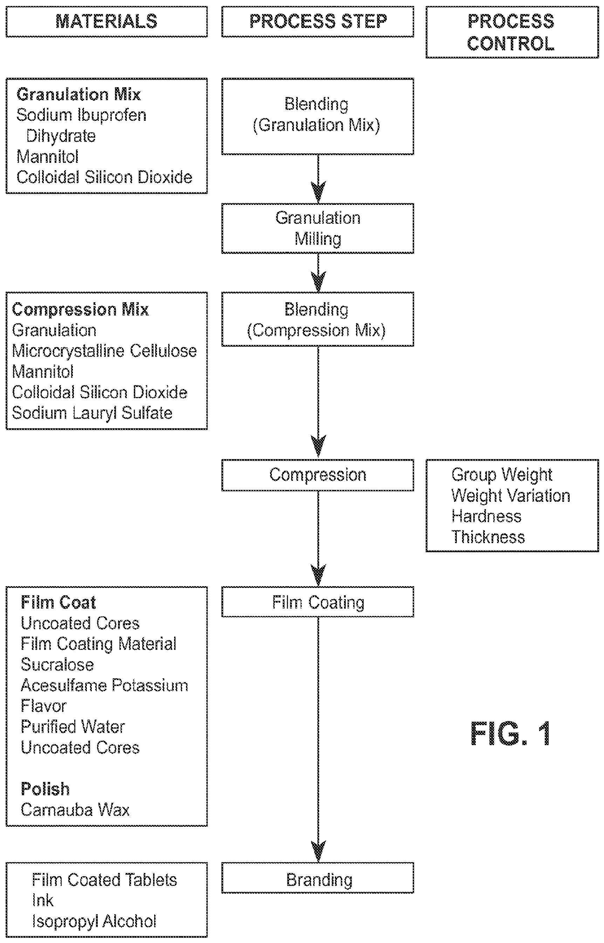

FIG. 1 shows a representative flow chart for the manufacture of 256.25 mg sodium ibuprofen tablets.
TABLE 3 summarizes a representative sodium ibuprofen formulation for manufacturing 256.25 mg coated tablets.
TABLE 4 summarizes a representative sodium ibuprofen formulation containing lactose for manufacturing 256.27 mg coated tablets.
TABLE 5 summarizes coating systems for manufacturing coated sodium ibuprofen Tablets and Caplets.
TABLE 6 summarizes roller compaction parameters for manufacturing sodium ibuprofen cores.
TABLE 7 summarizes sodium ibuprofen tablet compression data.
TABLE 8 summarizes sodium ibuprofen caplet compression data.
TABLE 9 summarizes hardness data for sodium ibuprofen coated tablets.
TABLE 10 summarizes hardness data for sodium ibuprofen coated tablets.
TABLE 11 summarizes in-process sodium ibuprofen tablet statistics.
TABLE 12 summarizes in-process sodium ibuprofen tablet hardness data.
TABLE 13 summarizes in-process sodium ibuprofen caplet hardness data.
TABLE 14 summarizes bulk friability data for a lactose containing sodium ibuprofen batch.
TABLE 15 (a), 3(b) and 3(c) show representative compositions of a sodium ibuprofen tablets. These formulations were used for the biostudy disclosed in Example 4.
TABLE 16 summarizes sodium ibuprofen medication study data.
TABLE 17 summarizes IBU Pharmacokinetic parameters
FIGURE 2: shows mean ibuprofen plasma concentration measurements from the Example 4 biostudy over time. Prototypes I - III correspond to formulations I - III (Tables 15(a) - 15(c)) from Example 10 respectively.
FIGURE 3 shows mean ibuprofen plasma concentration measurements from the Example 4 biostudy (Semi-Log scale) over time. Prototypes I - III correspond to formulations I - III (Tables 15(a) - 15(c)) from Example 10 respectively.
FIGURE 4 shows mean ibuprofen plasma concentration measurements from the Example 4 biostudy over the first two hours. Prototypes I - III correspond to formulations I - III (Tables 15(a) - 15(c)) from Example 10 respectively.
FIGURE 5 summarizes stability data at 25°C/60% relative humidity (RH) for lots of a composition of sodium ibuprofen.
FIGURE 6 summarizes stability data at 25°C/60% relative humidity (RH) and at 25° C/60% relative humidity (RH) S for lots of a composition of sodium ibuprofen.
FIGURE 7 summarizes stability data at 30°C/65% relative humidity (RH) and at 30° C/60% relative humidity (RH) S for lots of a composition of sodium ibuprofen.
FIGURE 8 summarizes dissolution data at 30°C/65% relative humidity (RH) and at 30° C/65% relative humidity (RH) S for a composition of sodium ibuprofen.
FIGURE 9 summarizes dissolution data at 40°C/75% relative humidity (RH) and at 30° C/60% relative humidity (RH) U for lots of a composition of sodium ibuprofen.
FIGURE 10 summarizes dissolution data at 40°C/75% relative humidity (RH) and at 40° C/75% relative humidity (RH) S for lots of a composition of sodium ibuprofen.
FIGURE 11 summarizes dissolution data at 25°C/60% relative humidity (RH) U for lots of a composition of sodium ibuprofen.
FIGURE 12 summarizes dissolution data at 25°C/60% relative humidity (RH) and at 25° C/60% relative humidity (RH) S for lots of a composition of sodium ibuprofen.

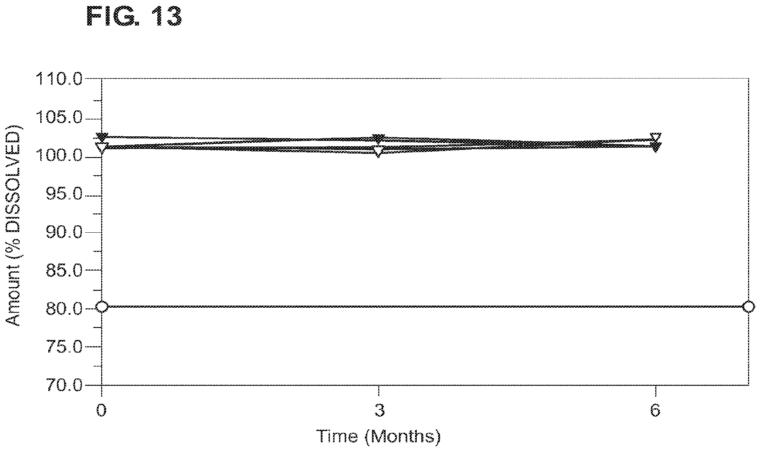

FIGURE 13 summarizes dissolution data at 30°C/65% relative humidity (RH) and at 30° C/60% relative humidity (RH) U for lots of a composition of sodium ibuprofen.

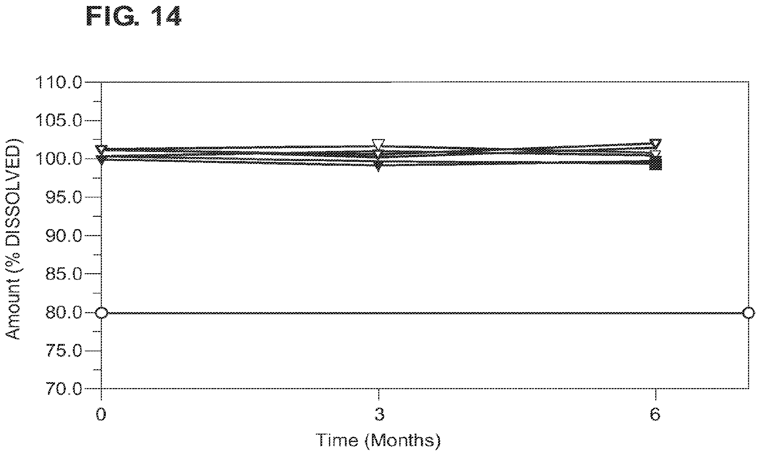

FIGURE 14 summarizes dissolution data at 30°C/65% relative humidity (RH) and at 30° C/65% relative humidity (RH) S for lots of a composition of sodium ibuprofen.

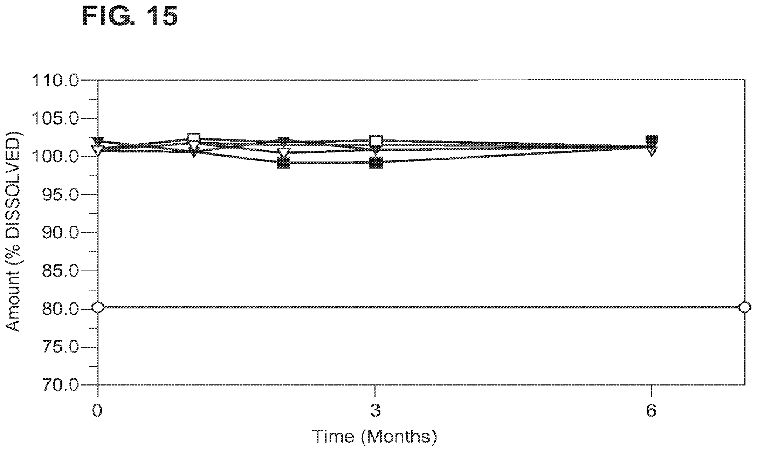

FIGURE 15 summarizes dissolution data at 40°C/75% relative humidity (RH) and at 30° C/60% relative humidity (RH) U for lots of a composition of sodium ibuprofen.

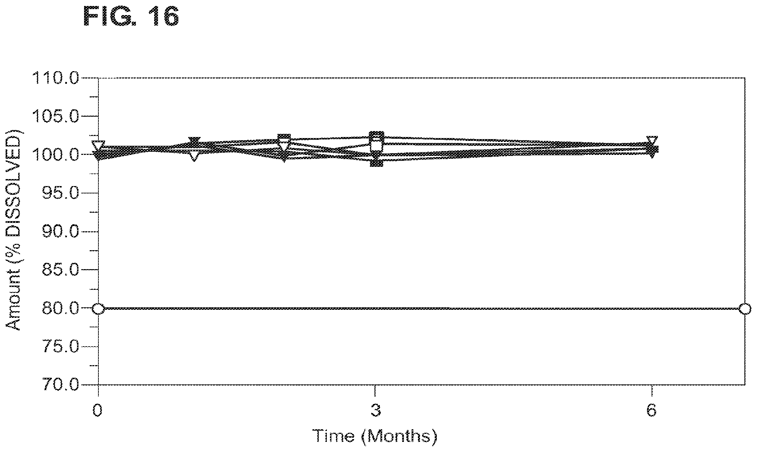

FIGURE 16 summarizes dissolution data at 40°C/75% relative humidity (RH) and at 40° C/75% relative humidity (RH) S for lots of a composition of sodium ibuprofen.

### DETAILED DESCRIPTION

The current invention provides sodium ibuprofen cores and corresponding coated tablet and caplets formed by compression. The ingredients and processes set forth herein allow for the manufacture of tablets and caplets with advantageous characteristics including rapid dissolution and excellent tablet strength. As used herein, the word "tablets" is intended to comprise tablets, caplets, capsule shaped tablets, pills or any other synonym thereof. Further, "tablet" refers to a pharmacological composition in the form of a small, essentially solid pellet of any shape. Tablet shapes maybe cylindrical, spherical, rectangular, capsular or irregular.

As used herein, the term "about" (or "approximately") means a particular value can have a range acceptable to those of skill in the art given the nature of the value and method by which it is determined.

Tablet strength is commonly measured by the diametrical compression test (also called the Brazilian test). See, e.g., Pharmaceutical Dosage Forms: Tablets. 3rd Edition. Vol. 1. Edited by Larry Augsburger and Stephen Hoag. pg 606. When a tablet fractures in a certain manner, the result may be assessed as the tensile strength. More generally, the peak load under which the tablet breaks is referred to as the crushing strength or crushing force. Newtons (N) are the SI units for this measurement, however, Strong Cobb Units (SCU) and Kiloponds (Kp) are sometimes used. Achieving an adequately strong tablet is important to avoid breakage during handling after compression, during film coating and when shipping the packaged product.

The tablets of the present invention also include one or more water soluble excipients. An excipient is any ingredient in the sodium ibuprofen core or coating except the active, and includes binders, diluents, disintegrants, flavoring agents, coloring agents, glidants, souring agents and sweeteners.

For the purposes of the present application, "binder" refers to one or more ingredients added before or during granulation to form granules and/or promote cohesive compacts during compression. Binders of the present invention include, at least, microcrystalline cellulose (MCC) and Mannitol. MCC is an ingredient that in water, with shear, forms a three-dimensional matrix comprised of millions of insoluble microcrystals that form an extremely stable, thixotropic gel. As a naturally occurring substance, it has proven to be stable, safe and physiologically inert. Microcrystalline cellulose (MCC) is known in the tableting art because of its unique compressibility and carrying capacity. It exhibits excellent properties as an excipient for solid dosage forms. It compacts well under a wide range of compression pressures, has high binding capability, and creates tablets that are extremely hard, stable, yet disintegrate rapidly. Other advantages include low friability, inherent lubricity, and the highest dilution potential of all binders. These properties make MCC particularly valuable as a filler and binder for formulations prepared by roller compaction, direct compression, and wet granulation. Mannitol, and preferably spray dried D-Mannitol with medium particle size, is also an excellent diluent-binder with good compressibility. Silicon Dioxide is also recognized and utilized herein for its binder characteristics. Those of ordinary skill will further appreciate that other binders could be added to formulate the compositions contemplated herein.

The tablet may also contain one or more glidant materials which improve the flow of the powder blend and minimize tablet weight variation. Glidants such as silicone dioxide may be used in the present invention. Those of ordinary skill will further appreciate that other glidants could be added or substituted to formulate the compositions contemplated herein.

Additionally, the tablets of the invention may include lubricants to facilitate ejection of the finished tablet from dies after compression and to prevent tablets from sticking to punch faces and each other. Two such ingredients contemplated herein are MCC and sodium lauryl sulfate. Further, a unique characteristic of sodium ibuprofen as an active ingredient is that it is itself a good lubricant. Those of ordinary skill will further appreciate that other lubricants could be added or substituted to formulate the compositions contemplated herein.

As used herein, the term "disintegrant" refers to one or more substances that encourage disintegration in water (or water containing fluid in vivo) of a pharmaceutical composition comprising the pharmaceutical formulations of the invention. In some embodiments, the disintegrant component comprises microcrystalline cellulose (MCC) plus one or more of crospovidone, alginic acid, sodium alginate, potassium alginate, calcium alginate, an ion exchange resin, carboxymethylcellulose, hydroxypropylcellulose, calcium silicate, a metal carbonate, sodium bicarbonate, calcium citrate, or calcium phosphate. Those of ordinary skill will further appreciate that other disintegrants could be added or substituted to formulate the compositions contemplated herein.

Diluents are herein referred to broadly as inactive ingredients or fillers that are added to tablets and caplets in addition to the active drug. Mannitol and MCC, along with their other characteristics are considered diluents. Those of ordinary skill will further appreciate that other diluents could be added or substituted to formulate the compositions contemplated herein.

Additionally, and optionally, other substances commonly used in pharmaceutical formulations can be included such as flavors (e.g., burnt sugar flavor, strawberry aroma, raspberry aroma, cherry flavor, magnasweet 135, key lime flavor, grape flavor, fruit extracts and prosweet), flavor enhancers and sweeteners (e.g., sucralose, aspartame, sodium saccharine, sorbitol, glucose, sucrose), souring agents (e.g. citric acid), dyes or colorants. Those of ordinary skill will further appreciate that other flavoring agents could be added or substituted to formulate the compositions contemplated herein.

As used herein, "having low sodium content" refers to pharmaceutically acceptable compositions providing a maximum daily sodium content of less than 140 mg/day. 21 CFR 201.64 "Labeling Requirements for Over-the-Counter Drugs" addresses the topic of sodium content in OTC drug products. A warning must appear if the maximum daily dose includes an amount of sodium above 140 mg daily. The labeling of OTC drug products intended for oral ingestion shall contain the following statement under the heading "Warning" (or "Warnings" if it appears with additional warning statements) if the amount of sodium present in the labeled maximum daily dose of the product is more than 140 milligrams: "Ask a doctor before use if you have [in bold type] [bullet]¹a sodium-restricted diet". One advantage of the invention disclosed herein is that such a warning is not required. It is contemplated that the total 140 mg/day of sodium may be provided broken up into multiple doses. For example, Example 2 discloses a tablet that includes 256.27 mg sodium ibuprofen. This equates to a dosage of 200 mg ibuprofen. With excipients that contain only a small amount of sodium, a single tablet or caplet per Example 2 would provide a sodium content of about 23 mg/dosage unit. Taking this tablet, an individual could take six unit doses and still be below both the maximum daily allowed OTC ibuprofen dose of 1200 mg/day and below the 140 mg/day sodium threshold. It is contemplated that a small amount of additional sodium can be present in the invented compositions, such as sodium lauryl sulfate (SLS) from Example 2, in accordance with the invention. However, the invented compositions still would provide a total sodium content of less than 140 mg/day.

The pharmaceutical industry employs various methods for compounding pharmaceutical agents in tablet formulations. With respect to the preparation of the ingredients, or a subset of the ingredients, for tableting, the preferred method for the compositions of the inventions disclosed herein is roller compaction. While having all the benefits a granulation process can provide such as improving material flow behavior and content uniformity, roller compaction offers unique advantages over wet granulation for moisture, solvent or heat (drying) sensitive compounds. In roller compaction, powder is fed to two counter-rotating rolls which draw the powder between the rolls due to friction and compact the powder. Roller compaction is seemingly a simple process but the fundamental mechanisms are complex due to a number of material properties and machine variables involved such as material flow properties, friction against roll surface, compressibility, compactibility, elastic properties, air permeability, roll surface, roll dimension, roll pressure, roll gap, roll speed, feed method and conditions (gravity or screw, screw design, vacuum or not) and feed pressure. In practice, roller compaction formulation and process development still largely relies on experience, trial-and-error and design of experiment. There is an apparent need to develop roller compaction product process development and scale-up methodology that is based on fundamental understanding but is also applicable to actual practice.

There are generally three controllable parameters in the roller compaction process: roll pressure, roll gap (or, when without gap control, ribbon thickness that can be controlled by feed screw speed), and roll speed. Because the consolidation of a powder blend into ribbons is the result of mechanical stress (normal and shear stresses) within the powder during roller compaction, all the parameters are studied by examining their correlation to the normal (compressive) stress and the shear stress.

Any method of forming a tablet of the invention into a desired shape which preserves the essential features thereof are within the scope of the invention.

Mixing and milling of tablet constituents during the preparation of a tablet composition may be accomplished by any method which causes the composition to become mixed to be essentially homogeneous.

Once tablet compositions are prepared, they may be formed into various shapes. In preferred embodiments, the tablet compositions are pressed into a shape. This process may comprise placing the tablet composition into a form and applying pressure to the composition so as to cause the composition to assume the shape of the surface of the form with which the composition is in contact. Parameters that are adjustable in most commonly used tablet presses can have great effect on the ultimate strength and stability of tablets contemplated by the inventions disclosed herein. These parameters, including tooling shape, pre-compression strength, compression force, turret speed are adjustable and effect tablet hardness and core defects including picking and sticking of primary particles that make up the core.

One advantage of the formulation of sodium ibuprofen, as compared to other sodium ibuprofen dosage forms, is that formulating with sodium ibuprofen allows for the formation of sodium ibuprofen cores having low sodium content and further provides tablets exhibiting improved physical stability, high core hardness and high core strength, coupled with excellent dissolution and bioavailability characteristics. Another advantage of the invented sodium ibuprofen composition is that ibuprofen preparations currently available on the market contain the active ingredient in the acid form, which is poorly soluble. Yet another advantage of the invented sodium ibuprofen cores and composition provide stable coated tablets/caplets having the necessary stability and dissolution profiles, including for example the required Tₘₐₓ. The invented sodium ibuprofen composition having an improved Tₘₐₓ in addition to other optimal parameters.

According to one embodiment, a pharmaceutical composition is provided comprising a core, said core comprising sodium ibuprofen, said composition having low sodium content. The expression "tablet core" indicates in the context of the present invention a tablet or caplet without sugar or film coat. The pharmaceutical composition is provided in the form of a tablet or caplet further comprising at least one coating. According to one embodiment, a pharmaceutical composition is provided comprising a core, said core comprising sodium ibuprofen, said composition having a ratio of sodium ibuprofen to total sodium content of about 11:1. The pharmaceutical composition of the invention comprises a coated core, said core containing sodium ibuprofen, said coated core having a sodium content of less than 23 mg/dosage unit. The pharmaceutical composition is further provided, wherein the Tₘₐₓ of ibuprofen obtained by a human taking two such cores is about 40 minutes or less.

According to one embodiment, the compositon comprises at least two binders including microcrystalline cellulose and mannitol. Examples of suitable binders are sugars such as saccharose, glucose, fructose and lactose, hexoses such as mannitol, xylitol, maltitol, sorbitol, hydrolysed or enzymatically splint starch such as maltodextrin, cyclodextrins such as P-and y- cyclodextrin and combinations thereof. The compositions of the invention comprise sodium ibuprofen dihydrate. The expression "sodium, ibuprofen hydrate" in the context of the present invention comprises all hydrates of sodium ibuprofen, including sodium ibuprofen di-hydrate, the sodium salt of racemic ibuprofen, as well as the sodium salts of the enantiomers S (+) -ibuprofen and R (-) -ibuprofen and of mixtures of these enantiomers. Preferably used are S (+) -sodium ibuprofen hydrate and, in particular, racemic sodium ibuprofen hydrate.

According to a separate embodiment, other salt forms of ibuprofen can be added to the invented core and corresponding composition. Typical examples include, but are not limited to, calcium ibuprofen, potassium ibuprofen, lysinate ibuprofen, arginate ibuprofen, carbonate salts of ibuprofen, phosphates salts, phosphates, hydrogen phosphates, oxides, hydroxides, citrates, tartrates, acetates or propionates, in particular basic sodium salts, trisodium citrate, disodium tartrate, dipotassium tartrate, magnesium oxide, calcium oxide, magnesium hydroxide, calcium hydroxide, magnesium carbonate, calcium carbonate, disodium hydrogen phosphate, dipotassium hydrogen phosphate, trisodium phosphate, tripotassium phosphate, tricalcium phosphate, sodium acetate, potassium acetate, sodium propionate etc., basic amino acids, such as lysine and arginine, and combinations thereof.

According to one embodiment a carbonate free core and corresponding composition is provided having a pH of 6.0 to 8.0. The cores and compositions lead to significantly supersaturated solutions in acidic medium, alding rapid resorption. In comparison to known ibuprofen medicines, the present invention therefore achieves more rapidly effective blood levels and concentrations at the site of effect, and thereby an accelerated onset of the analgesic effect, as well as a rapider achievement of the maximal blood levels and concentrations at the site of effect. Through numerous in vivo studies it has been verified that the maximal blood level is achieved with conventional ibuprofen, formulations only about 1.5 hours after administration. In contrast, maximal blood levels were already achieved after about 35 minutes with the tablets of this invention without disintegrant. The tablets of this invention therefore permit an especially rapid treatment of pains and lessen the danger that the patient takes another tablet as a result of a too slow onset of the analgesic effect.

According to one embodiment, the sodium ibuprofen tablets comprise sodium ibuprofen dihydrate that is present in an amount from 50 to 99.9% by weight, based on the weight of the pharmaceutical composition.

According to one embodiment, the sodium ibuprofen tablets comprise sodium ibuprofen dihydrate that is present in an amount of at least 60 to 90% by weight, based on the weight of the pharmaceutical composition.

According to one embodiment, the sodium ibuprofen tablets further comprise one or more additional excipients or fillers. The pharmaceutical composition is in the form of a coated tablet or coated caplet, the pH of an aqueous solution of the pharmaceutically acceptable composition ranging from 6.0 to 8.0.

According to one embodiment, the sodium ibuprofen tablets further comprise one or more pharmaceutically acceptable excipients that are present in an amount from 10 to 50% by weight, based on the pharmaceutical composition. Preferably water soluble excipients are used. Examples of preferably suitable excipients are sugars such as saccharose, glucose, fructose and lactose, hexoses such as mannitol, xylitol, maltitol, sorbitol, hydrolysed or enzymatically split starch such as maltodextrin, cyclodextrins such as P-and y- cyclodextrin, non-crosslinked (water soluble) polyvinylpyrrolidone, polyvinyl alcohols, polyethylene glycols, polypropylene glycols, alkali metal salts, alkaline earth metal salts and ammonium salts of organic or inorganic acids, in particular sodium, potassium, magnesium and calcium salts such as sodium chloride, potassium chloride, magnesium chloride, sodium sulphate, potassium sulphate, magnesium sulphate, trimagnesium dicitrate, tricalcium dicitrate, calcium lactate, calcium gluconate, calcium hydrogen phosphate and the like. Especially preferred excipients are hexoses such as sorbitol and mannitol, non-crosslinked polyvinylpyrrolidone, maltodextrin and sodium chloride, in particular water soluble, non-crosslinked polyvinylpyrrolidone, which is apparently also suitable to delay the precipitation of the ibuprofen in the stomach.

According to one embodiment the pharmaceutical composition comprises a coated core having at least one coating, comprising a sugar or film coating, in which all customary sugar and film coating materials are in principle suitable as coating materials. The thickness of the coat is not critical; however in general the proportion of the coat, based on the weight of the tablet core, is only about 1 to 10% by weight, including about 3 to 6% by weight. Suitable and exemplary coatings and coating materials are found in the Examples.

According to one embodiment, the sodium ibuprofen tablets/caplets comprise a hardness of greater than 30 N.

According to one embodiment, the sodium ibuprofen tablets/caplets comprise a hardness of greater than 40 N.

According to one embodiment, the sodium ibuprofen tablets/caplets comprise a hardness of greater than 80 N.

According to one embodiment, the sodium ibuprofen tablets/caplets comprise a hardness of greater than 90 N.

The tablets may also be coated with a rapidly dissolving water soluble polymeric film coat. Film coating involves the deposition of a thin, uniform, typically polymeric membrane to the substrate, usually by a spray technique. Advantages of the film coating process include minimal weight increase of the ultimate dosage form, reduction in processing times, and improved resistance to chipping. Optionally, the coating composition contains a flavoring agent in order to mask the taste and odor of the active ingredient. Further, polishing agents, such as canauba wax may be used as part of the coating process. Those of ordinary skill will further appreciate that other coating materials could be added or substituted to formulate the compositions contemplated herein. Further, methods other than film coating methods are contemplated herein.

### EXAMPLES

### Example 1

The following is an embodiment of a formulation contemplated by the inventors. A Sodium Ibuprofen Tablet, 200 mg is a round, beige film-coated tablet, printed with black ink, containing 256.25 mg of sodium ibuprofen dihydrate per dosage unit (equivalent to a 200 mg dose of ibuprofen).

Table 1 summarizes the composition of one sodium ibuprofen tablet drug product and the function of the excipients in the formulation.

**Table 1: Composition of Sodium Ibuprofen Tablet Drug Product**

| **Ingredient** | **Grade/Quality Standard** | **Unit Dose (mg/du)** | **Function** |
|---|---|---|---|
| Sodium Ibuprofen Dihydrate | N/A | 256.25 | Active Ingredient |
| Colloidal Silicon Dioxide | NF | 5.00 | Glidant, Binder |
| Mannitol | USP | 129 | Binder, Diluent |
| Microcrystalline Cellulose | NF | 39.6 | Binder, Disintegrant, Lubricant, Diluent |
| Sodium Lauryl Sulfate | NF | 0.500 | Lubricant, Wetting Agent |
| Film Coating Material (comprising hypromellose, copovidone and polyethylene glycol) | N/A | 15.8 | Cosmetic Tablet Film Coat |
| Acesulfame Potassium | NF | 0.0290 | Sweetening Agent |
| Sucralose | NF | 0.0900 | Sweetening Agent |
| Flavor (comprising ethyl alcohol and propylene glycol) | N/A | 0.229 | Flavoring Agent |
| Carnauba Wax | NF | 0.0425 | Polishing Agent |
| Opacode Black Ink | N/A | 0.09 | Branding |
| Purified Water | USP | N/A ^{a} | Coating dispersant |
| Isopropyl Alcohol | USP | N/A ^{a} | Ink Solvent |
| Total: | | 446 | |

| | | | |
|---|---|---|---|
| a. Essentially removed during processing. | | | |

### Example 2 (Comparative)

Another composition of a coated 200 mg dose of Sodium Ibuprofen Caplet containing lactose and the function of the excipients in the formulation is summarized in Table 2. A Sodium Ibuprofen Tablet, 200 mg is a round, beige film-coated tablet, printed with black ink, containing 256.27 mg of sodium ibuprofen dihydrate per dosage unit (equivalent to a 200 mg dose of ibuprofen).

**Table 2: Composition of Sodium Ibuprofen Tablet Drug Product**

| **Ingredient** | **Grade/Quality Standard** | **Unit Dose (mg/du)** | **Function** |
|---|---|---|---|
| Sodium Ibuprofen Dihydrate | N/A | 256.27 | Active Ingredient |
| Colloidal Silicon Dioxide | NF | 3.63 | Glidant, Binder |
| Mannitol | USP | 66.1 | Binder, Diluent |
| Fast Flo Lactose | NF | 85.0 | Binder, Diluent |
| Sodium Lauryl Sulfate | NF | 2.00 | Lubricant, Wetting Agent |
| Stearic Acid | | 2.00 | Lubricant |
| Film Coating Material (comprising hypromellose, talc and polyethylene glycol) | N/A | 14.6 | Cosmetic Tablet Film Coat |
| Acesulfame Potassium | NF | 0.029 | Sweetening Agent |
| Sucralose | NF | 0.090 | Sweetening Agent |
| Flavor | N/A | 0.229 | Flavoring Agent |
| Carnauba Wax | NF | 0.0425 | Polishing Agent |
| Purified Water | USP | N/A ^{a} | Coating dispersant |
| Total: | | 430 | |

| | | | |
|---|---|---|---|
| a. Essentially removed during processing | | | |

### Example 3

Following Example 1 is an embodiment of a larger scale batch formulation contemplated by the inventors. A batch of Sodium Ibuprofen Tablets was manufactured with a representative batch size of approximately 1.5 million tablets.

The manufacturing process for Sodium Ibuprofen is comprised of seven unit operations: weigh out, blending, roller compaction/milling, blending, compression, coating/polishing, and printing. The components of each unit operation are weighed out separately in the pharmacy.

Each sodium ibuprofen pre-blend was prepared by blending and layering screened sodium ibuprofen dihydrate, mannitol, and colloidal silicon dioxide into a bin. The contents of the bin were blended until uniform. The blend was then roller compacted and milled into granules using a roller compactor equipped with an integrated mill. After the roller compaction step, microcrystalline cellulose, mannitol, colloidal silicon dioxide, and sodium lauryl sulfate were screened and added to the bin to form the compression blend. The contents of the bin were blended until uniform. The compression blend was compressed into tablets on a rotary tablet press. At set up the following in-process testing was performed: average weight (421 to 439 mg, target 430 mg) and average hardness. In-process testing (average weight and average hardness) was performed throughout the compression stage to ensure the quality of the tablet cores being produced. After compression, the cores were coated with a sweetened film coat and a carnauba wax polish was applied in the film coating machine.

**Table 3: Representative Batch Formula for Sodium Ibuprofen Tablet (200 mg IBU)**

| **Ingredient** | **kg/batch** | **kg**/**bin** ^{a} |
|---|---|---|
| Granulation | | |
| Sodium Ibuprofen Dihydrate | 400.00 | 200.00 ^{b} |
| Mannitol | 31.20 | 15.6 ^{c} |
| Colloidal Silicon Dioxide | 4.68 | 2.34 ^{d} |
| Mannitol | 72.0 | 36.0 ^{e} |

| Compression Mix ^{f} | | |
|---|---|---|
| Sodium Ibuprofen Granulation | 507.89 | 253.95 |
| Mannitol | 97.56 | 48.78 |
| Microcrystalline Cellulose | 61.86 | 30.93 |
| Colloidal Silicon Dioxide | 3.12 | 1.56 |
| Sodium Lauryl Sulfate | 0.78 | 0.39 |

| Film Coating ^{g} | | |
|---|---|---|
| Ingredient | kg/ batch ^{h} | |
| Film Coating Material (comprising hypromellose, copovidone and polyethylene glycol) | 27.04 | |
| Acesulfame Potassium | 0.050 | |
| Flavor (comprising ethyl alcohol and propylene glycol) | 0.393 | |
| Purified Water | 110.6 ⁱ | |
| Sucralose | 0.155 | |

| Polishing | | |
|---|---|---|
| Ingredient | kg/ batch | Kg/bin ^{a} |
| Carnauba Wax | 0.060 | 0.030 |

| Branding | | |
|---|---|---|
| Ingredient | kg/ batch | |
| Ink | 3.00 ^{j} | |
| Isopropyl Alcohol | 3.00 ^{i,j, k} | |

| | | |
|---|---|---|
| a. Two bins of material consist of one batch. b. Sodium Ibuprofen Dihydrate should be divided into 50.0 kg aliguots (four portions) c. Mannitol should be divided into three aliquots of 5.20 kg for use in Mannitol/ Collodial Silicon Dioxide mixes. d. Collodial Silicon Dioxide should be divided into three aliquots of 0.78 kg for use in Mannitol/Collodial Silicon Dioxide mixes. e. Mannitol should be divided into three aliquots of 12.0 kg. f. If the yield of Granulation (% Theoretical Yield) is out of the specified range (97.0 - 102.0%), the compression mix components will be calculated based on the actual yield. g. Excess coating suspension is prepared to allow for priming of lines; coating suspension is 20% solids. h. One tank of film coating solution is prepared to coat the batch (2 bins). i. Does not appear in the final dosage form, essentially removed during processing. j. Excess ink and alcohol is dispensed for set-up. Amounts include overages that may not be used during processing. k. Alcohol will be used to thin the ink, as needed. | | |

### Example 4 (Comparative)

Following Example 3 is an embodiment of a larger scale batch formulation contemplated by the inventors. A batch of coated Sodium Ibuprofen Tablets containing lactose was manufactured with a representative batch size of approximately 1 million tablets.

**Table 4: Representative Batch Formula for Sodium Ibuprofen Tablet (200 mg IBU)**

| **Ingredient** | **kg/batch** |
|---|---|
| Granulation | |
| Sodium Ibuprofen Dihydrate | 174.0 |
| Mannitol | 44.9 |
| Colloidal Silicon Dioxide | 1.1 |
| Compression Mix | |
| Sodium Ibuprofen Granulation | 220.0 |
| Lactose | 57.7 |
| Colloidal Silicon Dioxide | 1.4 |
| Sodium Lauryl Sulfate | 1.4 |
| Stearic Acid | 1.4 |
| Film Coating | |

| **Ingredient** | **g/batch** |
|---|---|
| Cores | 12,000 |
| Film Coating Material (comprising hypromellose, talc and polyethylene glycol) | 598.1 |
| Acesulfame Potassium | 1.19 |
| Flavor | 9.38 |
| Purified Water¹ | 3,481.9 |
| Sucralose | 3.69 |
| Polishing | |
| Carnauba Wax | 1.258 |

| | |
|---|---|
| 1. Does not appear in the final dosage form, essentially removed during processing | |

### EXAMPLE 5

Other examples of coated sodium ibuprofen cores for tablet and caplet products were manufactured with the following coating systems summarized in Table 5.

**Table 5. Coating Systems for Sodium Ibuprofen Tablet/Caplet Cores Used During Process Development**

| **Coating System** | **Qualitative List of Ingredients** |
|---|---|
| C1 | Hypromellose 6cP |
| | Hypromellose 3cP |
| | Titanium Dioxide |
| | Talc |
| | Polyethylene Glycol 8000 |
| | Polyethylene Glycol 400 |
| | Iron Oxides |
| C2 | Hypromellose |
| | Hydroxypropyl Cellulose |
| | Glycerine |
| | Titanium Dioxide |
| | Iron Oxides |
| C3 | Hypromellose |
| | Copovidone |
| | Polyethylene Glycol |
| | Medium Chain Triglycerides |
| | Titanium Dioxide |
| | Iron Oxides |
| C4 | Hypromellose |
| | Copovidone |
| | Polyethylene Glycol |
| | Medium Chain Triglycerides |
| | Titanium Dioxide |
| | Iron Oxides |

### Example 6

### Description of Manufacturing Process And Process Controls

A flow chart of the manufacture of Sodium Ibuprofen Tablets, 200 mg is presented in Figure 1.

### Example 7

The following manufacturing procedure describes the steps in the manufacturing process for Sodium Ibuprofen Tablets, 200 mg.

### Manufacturing Process

The following manufacturing procedure describes the steps in the manufacturing process for the drug product Sodium Ibuprofen tablets, 200 mg.

### Weight Out

The indicated quantities of each component were weighed and placed into separate, appropriately labeled containers.

### Blending (Sodium ibuprofen Pre-Blend)

Blending of the granulation mix was performed in a bin blender. One batch consists of ten bins. The following procedure was used to charge each of the bins:
1) Prepared mixes of mannitol and colloidal silicon dioxide.
2) Screened all ingredients through a #20 mesh screen into suitable containers, keeping all ingredients separate.
3) Placed materials in the bin by alternating sodium ibuprofen dihydrate, mannitol, and mannitol/colloidal silicon dioxide mix aliquots until all material was in the bin.

Blended materials for 3 to 15 minutes at 17 rpm ± 1 rpm. Repeat blending steps for each of the ten bins.

### Roller Compaction/Milling

The pre-blend was fed into the roller compactor directly from the bin used in blending. Maintain the roller compaction parameters listed in Table 6 to produce acceptable ribbons.

**Table 6: Roller Compaction Parameters**

| **Parameter** | **Range** |
|---|---|
| Press Force (kN/cm) | 2.0 - 6.0 |
| Roll Gap (mm) | 2.0 - 4.5 |

Following roller compaction, processed the ribbons through an integral, oscillating mill equipped with a 1.5 mm screen. Collected the milled material in suitable containers.

### Blending (Compression Mix)

Blending of the compression mix is performed in a bin blender for each of the bin equivalences of granulation. The following procedure is used to charge each of the bins:
1) Using an appropriate container, the colloidal silicon dioxide was combined with microcrystalline cellulose.
2) Screened the colloidal silicon dioxide/microcrystalline cellulose mix, sodium lauryl sulfate, and mannitol through a #20 mesh screen.
3) To the sodium ibuprofen granulation in the bin was added the screened colloidal silicon dioxide/microcrystalline cellulose mix, sodium lauryl sulfate, and mannitol.

Blended materials for 9 to 18 minutes at 17 rpm ± 1 rpm.
This procedure was repeated for each of the ten bins constituting one batch.

### Compression

Using a rotary tablet press equipped with round or capsule-shaped tooling, compressed the compression mix as the caplet core. Average weight was measured to ensure content uniformity. Deviations from the target weight were corrected by adjusting the fill depth. Average hardness was measured to ensure performance and robustness of the core. Collected tablets in suitable storage containers after passing through a de-duster and metal detector. Exemplary compression parameters for coated sodium ibuprofen tablets and caplets are summarized in Tables 7 and 8. It is contemplated that a broader range of such compression parameters are usefully employed in accordance with the invention.

**Table 7. Sodium Ibuprofen Tablet Compression Data**

| | Target (triple tip) | Target (single tip) | Preferred range (triple tip) | Preferred range (single tip) |
|---|---|---|---|---|
| Pre Compression (kN) | 4.2-4.9 | 1.5 | 2.0-6.3 | 1.0-1.9 |
| Main Compression (kN) | 40 | 14 | 26-48 | 12-20 |
| Turret Speed (rpm) | 20 | 20 | 10-20 | 10-30 |

**Table 8. Sodium Ibuprofen Caplet Compression Data**

| | Target (single tip) | Preferred range (single tip) |
|---|---|---|
| Pre Compression (kN) | 1.5 | 1.1-1.8 |
| Main Compression (kN) | 17 | 10-21 |
| Turret Speed (rpm) | 20 | 10-20 |

Compression Forces and Hardness Data for Representative Sodium Ibuprofen Tablets and Caplets is summarized in Tables 9-13.

**Table 9. In-Process Weight, Thickness, Hardness - Batch from Example 2**

| **Time Point** | **Actual Time (hh:mm)** | **Averages of 10 Tablet Cores** | | |
|---|---|---|---|---|
| | | **Weight, g** | **Hardness, N^{a}** | **Thickness, mm^{a}** |
| 1 | 16:01 | 0.4117 | 29.4 | 5.5499 |
| 2 | 16:05 | 0.4046 | 35.0 | 5.49148 |
| 3 | 16:23 | 0.4174 | 43.4 | 5.6007 |
| 4 | 16:25 | 0.4142 | 32.9 | 5.58038 |
| 5 | 16:40 | 0.4182 | 44.8 | 5.59308 |
| 6 | 16:42 | 0.415 | 30.1 | 5.57784 |
| 7 | 16:59 | 0.415 | 51.1 | 5.59054 |
| 8 | 17:01 | 0.4133 | 42.7 | 5.56006 |
| **Mean** | | **0.4144** | **38.7** | **5.5680** |
| **SD** | | **0.0022** | **7.9** | **0.0353** |
| **%RSD** | | **0.5** | **20.4** | **0.6** |

| | | | | |
|---|---|---|---|---|
| a. Hardness was converted from scu to N and thickness was converted from in to mm. | | | | |

**Table 10. In-Process Weight, Thickness, Hardness - Batch from Example 2**

| **Time Point** | **Actual Time (hh:mm)** | **Averages of 10 Tablet Cores** | | |
|---|---|---|---|---|
| | | **Weight, g** | **Hardness, N^{a}** | **Thickness, mm**^{a} |
| 1 | 14:46 | 0.4121 | 37.1 | 6.02463 |
| 2 | 14:43 | 0.4129 | 32.9 | 6.03809 |
| 3 | 15:00 | 0.4146 | 37.8 | 5.97408 |
| 4 | 15:04 | 0.4182 | 35.0 | 5.99796 |
| 5 | 15:16 | 0.4128 | 38.5 | 5.95554 |
| 6 | 15:19 | 0.4151 | 37.8 | 5.97484 |
| 7 | 15:31 | 0.4127 | 46.2 | 5.94716 |
| 8 | 15:34 | 0.4169 | 35.0 | 6.00380 |
| **Mean** | | **0.4137** | **37.6** | **5.5661** |
| **SD** | | **0.0042** | **4.0** | **0.0173** |
| **%RSD** | | **1.0** | **10.6** | **0.3** |

| | | | | |
|---|---|---|---|---|
| a. Hardness was converted from scu to N and thickness was converted from in to mm. | | | | |

**Table 11. In-Process Tablet Statistics for Tablet Cores from Examples 15a-c**

| **Batch** | **Example 15a^{a}** | | | **Example 15b^{a}** | | | **Example 15c^{a}** | | |
|---|---|---|---|---|---|---|---|---|---|
| | **Weight (g)** | **Hardness (N)** | **Thickness (mm)** | **Weight (g)** | **Hardness (N)** | **Thickness (mm)** | **Weight (g)** | **Hardness (N)** | **Thickness (mm)** |
| Min | 4.44 | 90.4 | 5.93 | 4.44 | 89.9 | 5.94 | 4.44 | 84.1 | 5.95 |
| Max | 4.57 | 112.8 | 6.04 | 4.53 | 105.8 | 6.02 | 4.54 | 97.4 | 6.00 |
| Mean | 4.49 | 101.7 | 5.98 | 4.48 | 96.6 | 5.97 | 4.47 | 90.6 | 5.98 |
| St Dev | 0.04 | 5.3 | 0.03 | 0.03 | 5.6 | 0.03 | 0.03 | 3.7 | 0.02 |
| %RS D | 0.86 | 5.2 | 0.55 | 0.71 | 5.8 | 0.50 | 0.64 | 4.0 | 0.27 |

| | | | | | | | | | |
|---|---|---|---|---|---|---|---|---|---|
| a. Hardness was converted from scu to N and thickness was coverted from in to mm | | | | | | | | | |

**Table 12. Mean Statistics for In-Process Average Hardness for Tablet Core Lots from Example 3**

| **Average 80 to 200 N for 10 Tablet Core Lots** | | | |
|---|---|---|---|
| **Tablet Type (Statistic)** | **Lot 1** | **Lot 2** | **Lot 3** |
| Minimum | 93 | 96 | 99 |
| Maximum | 108 | 106 | 108 |
| Mean | 100 | 102 | 103 |
| %RSD | 3.58 | 2.26 | 1.99 |

**Table 13. Mean Statistics for In-process Average Hardness for Caplet Lots from Example 3**

| **Average 90 to 200 N for 10 Caplet Core Lots** | | | |
|---|---|---|---|
| **Caplet Type (Statistic)** | **Lot 1** | **Lot 2** | **Lot 3** |
| Minimum | 108 | 109 | 108 |
| Maximum | 118 | 120 | 121 |
| Mean | 114 | 114 | 114 |
| %RSD | 1.89 | 1.78 | 2.13 |

### Suspension Preparation

1) Added colored film coating material to water and mixed for at least 30 minutes.
2) Added the sweeteners and one or more flavor agents to the suspension and continue mixing for at least 15 minutes.

### Film Coating

1) Transferred a quantity of caplet or tablet cores to an appropriately sized coating pan. Using the coating system prepared, applied the calculated amount of suspension to the caplet or tablet bed.

Upon completion of the coating suspension application, applied carnauba wax screened through a mesh screen to the caplet or tablet bed.
2) Tumbled caplets or tablet to distribute carnauba wax
3) Discharged the caplets or tablet from the coater into appropriate containers.

### Printing

Print caplets or tablet on one side with black ink, diluted as needed with isopropyl alcohol, at a speed that produces acceptable print quality, using an offset printer.

### Packaging

Tablets or caplets were packaged by conventional techniques.

### Example 8

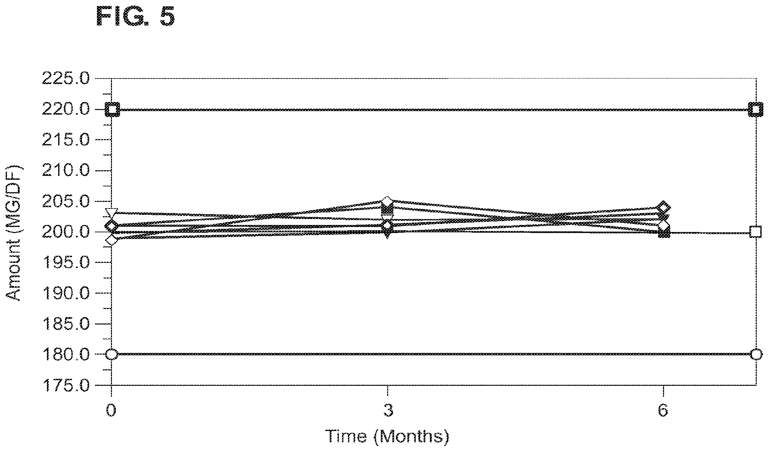

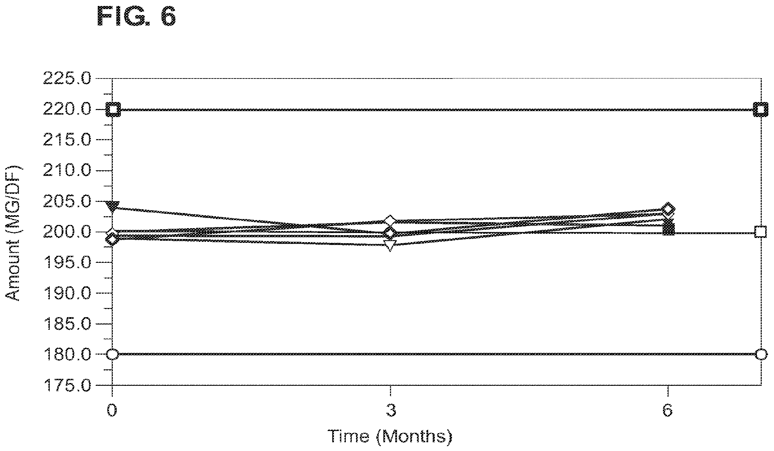

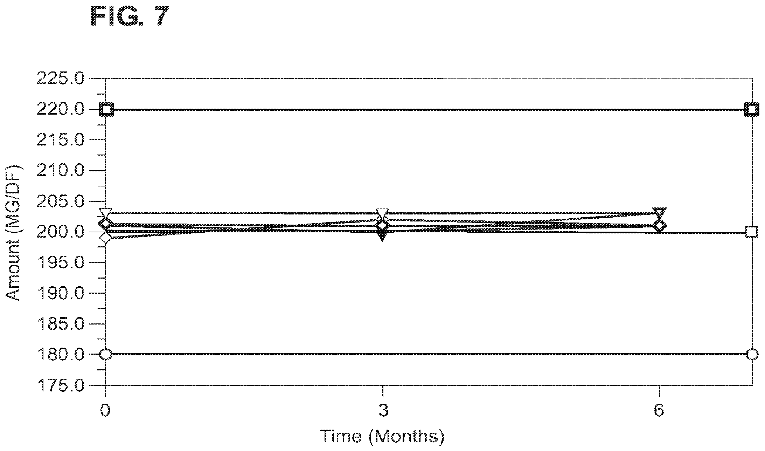

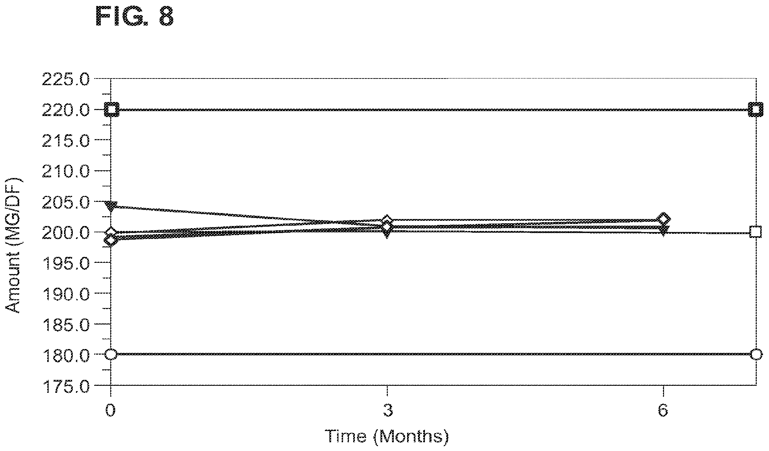

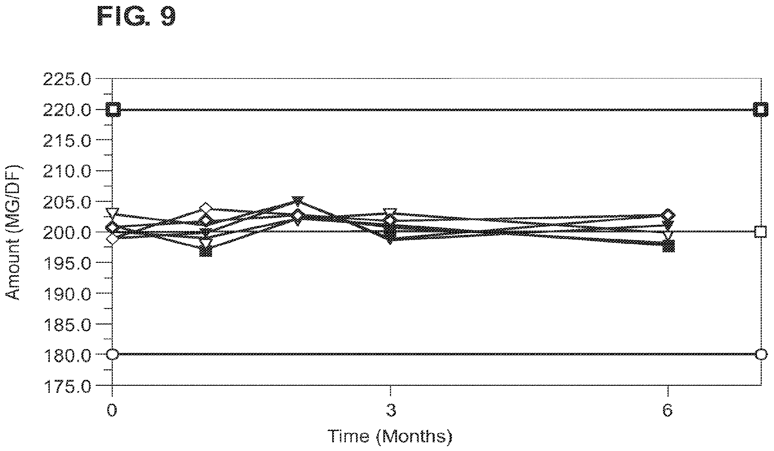

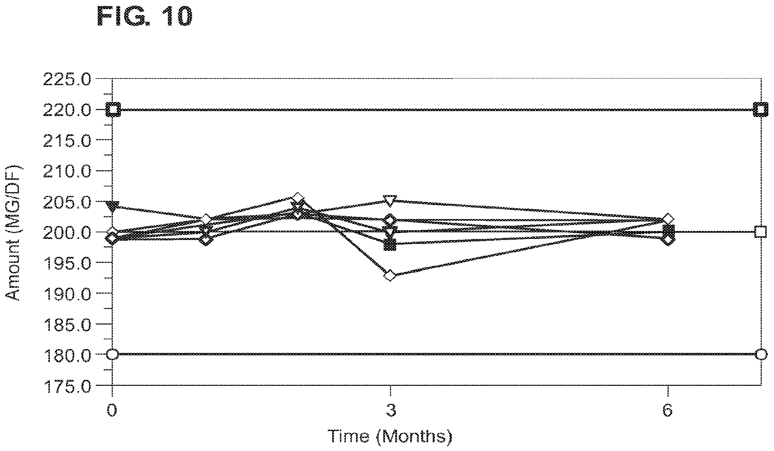

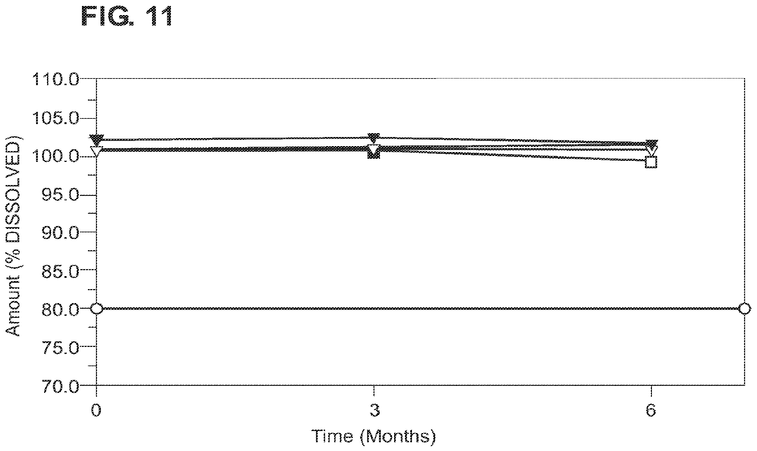

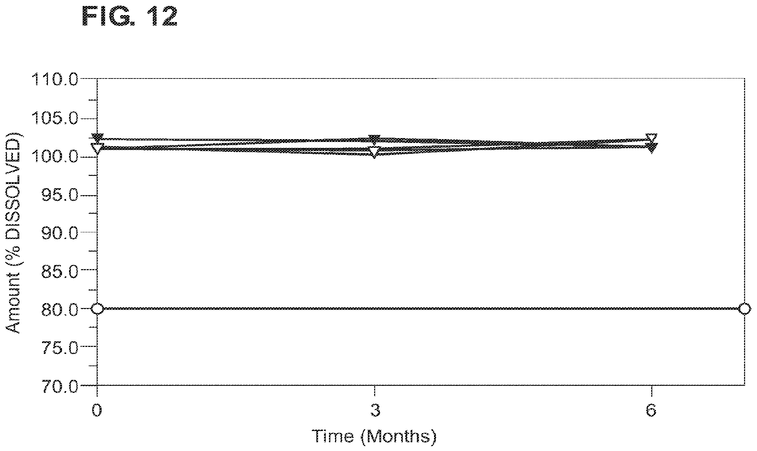

Friability Data for Sodium Ibuprofen coated tablets from Example 1.
Stability and Dissolution Studies of the Sodium Ibuprofen compositions are summarized in Figures 5-12.

### Example 9

Friability Data are summarized for a coated Sodium Ibuprofen Coated Compositions from Example 15(a) are summarized in Table 14. Exemplary Bulk friability Data for a Sodium Ibuprofen Batch Containing Lactose is 0.47%. Friability was tested after specified revolutions according to USP <1216> tablet friability testing.

**Table 14. Friability and Disintegration Data from Example 15(a)**

| **Friability and Disintegration 3 Formulation Lots from 15(a)** | | | |
|---|---|---|---|
| Sample | Friability after 100 revs | Friability after 500 revs | Disintegration time (min) |
| 1R | 0.13% | 0.54% | 4.42 |
| 1L | 0.11% | 0.55% | 4.73 |
| 2R | 0.13% | 0.50% | 4.31 |
| 2L | 0.11% | 0.57% | 4.97 |
| 3R | 0.13% | 0.58% | 4.28 |
| 3L | 0.08% | 0.50% | 4.84 |
| 4R | 0.02% | 0.53% | 4.23 |
| 5R | 0.11% | 0.63% | 3.68 |
| 6R | 0.11% | 0.49% | 4.78 |
| 6L | 0.13% | 0.51% | 5.11 |
| 7R | 0.10% | 0.53% | 5.04 |
| 7L | 0.06% | 0.55% | 5.48 |
| 8R | 0.14% | 0.58% | 4.91 |
| 8L | 0.22% | 0.52% | 5.16 |
| 9R | 0.18% | 0.27% | 4.94 |
| 9L | 0.16% | 0.59% | NA |
| 10R | 0.16% | 0.60% | 4.76 |
| 10L | 0.19% | 0.57% | NA |
| 11R | 0.13% | 0.49% | 4.70 |
| 11L | 0.12% | 0.52% | NA |
| 12R | 0.14% | 0.52% | 5.07 |
| 12L | 0.16% | 0.54% | NA |

### Example 10

A Pilot Study to Compare the Absorption of Sodium Ibuprofen Prototype Tablets

This pilot study evaluated the absorption profile of three different sodium ibuprofen prototype tablets compared to a currently marketed ibuprofen product (hereinafter "reference standard").

The objective of this study was to compare the rate and extent (up to 6 hours) of ibuprofen absorption from sodium ibuprofen prototype tablets to the reference standard.

### Overall Study Design and Plan Description

This was a single-dose, randomized, open-label, in-patient, four-way crossover study. Sixteen healthy male and female subjects (approximately equal numbers of each gender) were planned to be enrolled to ensure that at least 12 subjects completed the study. The subjects were randomly assigned to 1 of 4 dosing sequences and received a 400 mg dose of each ibuprofen formulation following an overnight fast in each of the study periods. Dosing for each study period was separated by at least 48 hours. Eighteen blood samples (3 mL each) were collected into sodium heparin tubes from each subject for the analysis of racemic ibuprofen over 6 hours during each of the four study periods. A total of approximately 216 mL of blood was drawn from each subject during the study (excluding approximately 30 mL of blood required for safety and pregnancy evaluations). Subjects were housed on-site for the duration of the study.

### Identity of Investigational Product

Tables 15(a) through 15(c) set forth prototypes I - III used for the biostudy.

**Table 15(a): Formulation I**

| This prototype was manufactured into round, brown tablets. The uncoated weight of the core was 450 mg. | |
|---|---|
| **Roller Compaction** | |
| **Component Name** | **mg/tab** |
| Sodium ibuprofen | 256.25 |
| Silicon Dioxide Colloidal | 1.63 |
| Mannitol | 66.12 |

| **Compression Mix** | |
|---|---|
| **Component Name** | **mg/tab** |
| Stearic Acid | 2.0 |
| Microcrystalline Cellulose | 60.0 |
| Silicon Dioxide Colloidal | 2.0 |
| Sodium Lauryl Sulfate | 2.0 |
| Mannitol | 60.0 |

| **Coating** | |
|---|---|
| **Component Name** | **mg/tab** |
| Film Coating Material (comprising hypromellose, polyethylene glycol and coloring agents) | 15.75 |
| Purified Water USP | N/A |
| Carnauba Wax #1 | 0.0425 |
| Acesulfame Potassium | 0.029 |
| Sucralose Micronized Powder | 0.090 |
| Flavoring Agent (comprising ethyl alcohol, natural and artificial flavors and propylene glycol) | 0.229 |

**Table 15(b): Formulation II**

| This prototype was manufactured into round, brown tablets. The uncoated weight of the core was 450 mg. | |
|---|---|
| **Roller Compaction** | |
| **Component Name** | **mg/tab** |
| Sodium ibuprofen | 256.25 |
| Silicon Dioxide Colloidal | 1.63 |
| Mannitol | 66.12 |

| **Compression Mix** | |
|---|---|
| **Component Name** | **mg/tab** |
| Stearic Acid | 2.0 |
| Microcrystalline Cellulose | 30.0 |
| Silicon Dioxide Colloidal | 2.0 |
| Sodium Lauryl Sulfate | 2.0 |
| Mannitol | 90.0 |

| **Coating** | |
|---|---|
| **Component Name** | **mg/tab** |
| Film Coating Material (comprising hypromellose, polyethylene glycol and coloring agents) | 15.75 |
| Purified Water USP | N/A |
| Carnauba Wax #1 | 0.0425 |
| Acesulfame Potassium | 0.029 |
| Sucralose Micronized Powder | 0.090 |
| Flavoring Agent (comprising ethyl alcohol, natural and artificial flavors and propylene glycol) | 0.229 |

**Table 15(c): Formulation III**

| Formulation III was manufactured into round brown tablets. Uncoated weight of th tablets was 450 mg. | |
|---|---|
| **Roller Compaction** | |
| **Component Name** | **mg/du** |
| Sodium ibuprofen | 256.25 |
| Silicon Dioxide Colloidal | 1.63 |
| Mannitol | 59.64 |
| Crospovidone | 6.48 |

| **Compression Mix** | |
|---|---|
| **Component Name** | **mg/du** |
| Stearic Acid | 2.0 |
| Microcrystalline Cellulose | 58.74 |
| Silicon Dioxide Colloidal | 2.0 |
| Sodium Lauryl Sulfate | 2.0 |
| Mannitol | 58.74 |
| Crospovidone | 2.52 |

| **Coating** | |
|---|---|
| **Component Name** | **mg/du** |
| Film Coating Material (comprising hypromellose, polyethylene glycol and coloring agents) | 15.75 |
| Purified Water USP | N/A |
| Carnauba Wax #1 | 0.0425 |
| Acesulfame Potassium | 0.029 |
| Sucralose Micronized Powder | 0.090 |
| Flavoring Agent (comprising ethyl alcohol, natural and artificial flavors and propylene glycol) | 0.229 |

- **Treatment A:** 2 x sodium ibuprofen 256 mg prototype tablets formulation I (equivalent to 400 mg ibuprofen) at 0 hours;
- **Treatment B:** 2 x sodium ibuprofen 256 mg prototype tablets formulation II (equivalent to 400 mg ibuprofen) at 0 hours;
- **Treatment C:** 2 x sodium ibuprofen 256 mg prototype tablets formulation III (equivalent to 400 mg ibuprofen) at 0 hours;
- **Treatment D, reference:** 2 x reference standard 200 mg (total dose = 400 mg) at 0 hours.

All treatments were administered under fasting conditions.

### Treatments Administered

**Table 16: Study Medication**

| **Drug** | **Per Unit** | **Per Dose** |
|---|---|---|
| Sodium ibuprofen prototype tablet formulation I | Sodium ibuprofen 256 mg (equivalent to ibuprofen 200 mg) | 2 tablets orally |
| Sodium ibuprofen prototype tablet formulation II | Sodium ibuprofen 256 mg (equivalent to ibuprofen 200 mg) | 2 tablets orally |
| Sodium ibuprofen prototype tablet formulation III | Sodium ibuprofen 256 mg (equivalent to ibuprofen 200 mg) | 2 tablets orally |
| Reference Standard | Solubilized ibuprofen 200 mg | 2 liquid capsules orally |

### Bioanalytical Methodology

Plasma samples were analyzed for racemic IBU using a validated method of high performance liquid chromatography with tandem mass spectrometry/mass spectrometry (HPLC MS/MS) detection.

The following PK parameters were derived: AUCL, Cₘₐₓ, Ln AUCL, Ln Cₘₐₓ, Tₘₐₓ, T_{mec} (time to reach a plasma concentration of 6.4mcg/mL), T₂₀ (time to reach a plasma concentration of 20mcg/mL) and T_{lag} (time delay between drug administration and the onset of absorption).

### Pharmacokinetic Comparisons

The following pairs of comparisons were evaluated:
- Sodium ibuprofen prototype tablet formulation I (Treatment A) vs. Reference Standard (Treatment D)
- Sodium ibuprofen prototype tablet formulation II (Treatment B) vs. Reference Standard (Treatment D)
- Sodium ibuprofen prototype tablet formulation III (Treatment C) vs. Reference Standard (Treatment D)

### Statistical Analysis

AUCL and Cₘₐₓ data, both log transformed and untransformed, were analyzed for differences between treatments using an analysis of variance (ANOVA) with effects for gender, subject (gender), period, treatment, and treatment-by-gender interaction. The treatment-by-gender interaction was to be retained in the final model if it was significant (at 0.10 level). The gender effect was tested using subject (gender) as the error term, and using sequential (type 1) sums of squares.

A total of 17 subjects (8 (47%) males and 9 (53%) females), 23-44 years of age, participated in the trial. The average age, and body mass index of the population were 30.6 years (range 23-44 years) and 24.3 kg/m² (range 20.0 - 28.0 kg/m²). Eleven (64.7%) of the subjects were White, followed by 3 (17.7%) Black, 2 (11.8%) Asian, and 1 (4.9%) classified as 'Other' race. Eight (47.1%) subjects were of Hispanic ethnicity.

### PHARMACOKINETIC RESULTS

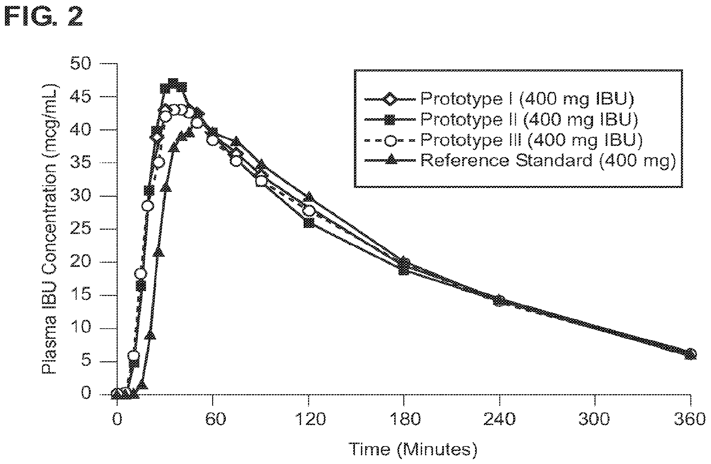

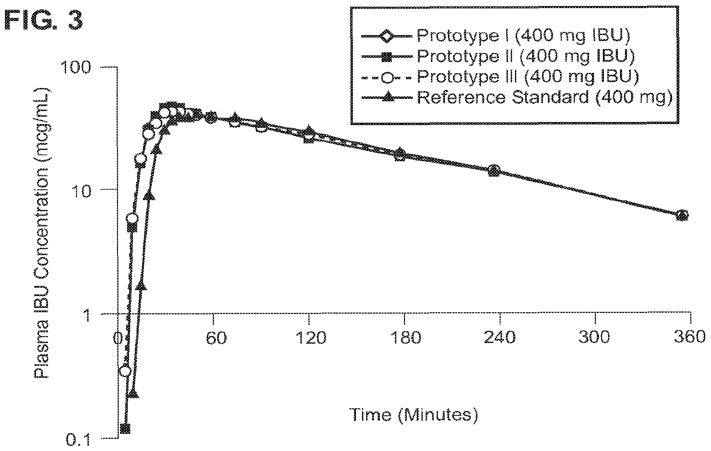

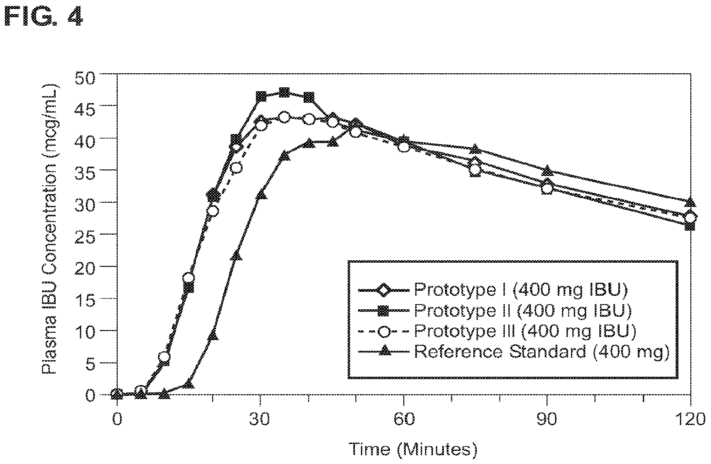

Individual subject concentration data at each sampling time, as well as the summary statistics for the ibuprofen plasma concentration at each sampling time are graphed below. The mean plasma concentration curves are illustrated in Figure 2 (linear scale) and Figure 3 (semi-log scale) below. The mean plasma concentration curves (linear scale) up to 2 hours after dosing are shown in Figure 4.

### Pharmacokinetic Data

The key results are summarized in Table 17, below. Each of the three prototypes was bioequivalent to the Reference Standard with respect to both extent (AUCL) up to 6 hours, and rate (Cₘₐₓ) of ibuprofen absorption, with the confidence limits for each ratio of the test vs reference formulation contained well within the pre-defined range (75.0-133.3%), as well as the conventional range (80-125%) for bioequivalence. All three formulations were rapidly absorbed (Figure 4), and reached their respective peak concentrations (Tₘₐₓ) within 40 minutes on average, somewhat faster relative to The Reference Standard, which showed a mean Tₘₐₓ of -52 minutes. The three prototypes reached T_{mec} (time to a plasma concentration of 6.4 mcg/mL) within 12 minutes of dosing and T₂₀ (time to a plasma concentration of 20mcg/mL) within 18.2 minutes of dosing, faster than the respective times for the Reference Standard of approximately 22 minutes and 29 minutes.

Overall, prototype II formulation exhibited the fastest PK profile with shortest times to relevant plasma concentration thresholds (Tₘₐₓ, T_{mec} and T₂₀) and the highest Cₘₐₓ; however, the PK profiles of the other two prototypes were also promising, and were similar to that of prototype II.

The key results are summarized in Table 17 below.

**Table 17: Summary of Results - IBU Pharmacokinetic Parameters (Mean, Standard Deviation, and 90% Confidence Intervals)**

| **Treatment** | **AUCL (mcg.h/mL)** | **Cₘₐₓ (mcg/mL)** | **Tₘₐₓ (min)** | **T_{mec} (min)** | **T₂₀ (min)** |
|---|---|---|---|---|---|
| A: IBU prototype I | 125.80(21.5) | 47.41(8.6) | 38.75(10.8) | 11.36(4.3) | 17.74(6.4) |
| B: IBU prototype II | 123.98(20.0) | 49.58(7.8) | 32.76(6.1) | 10.77(4.4) | 16.31(5.0) |
| C: IBU prototype III | 123.44(16.5) | 47.06(9.0) | 36.70(12.3) | 11.72(5.3) | 18.16(7.6) |
| D: Ref. Standard | 121.52(18.8) | 47.61(8.9) | 52.36(16.7) | 22.18(8.5) | 28.94(12.6) |
| A/D Ratio^ (%) | 103.26 | 99.73 | | | |
| 90% CI^ | 100.7-105.9 | 93.0-106.9 | | | |
| B/D Ratio^ (%) | 101.93 | 104.62 | | | |
| 90% CI^ | 99.4-104.6 | 97.6-112.2 | | | |
| C/D Ratio^ (%) | 101.87 | 98.74 | | | |
| 90% CI^ | 99.3-104.5 | 92.1-105.8 | | | |

| | | | | | |
|---|---|---|---|---|---|
| ^Based on fitted log-transformed parameters. Note: Each formulation contained a molar equivalent of 400mg of ibuprofen. | | | | | |

### Overall Pharmacokinetic Conclusions

All three prototypes were bioequivalent to the Reference Standard with respect to both extent (AUC) up to 6 hours, and rate (Cₘₐₓ) of ibuprofen absorption. Confidence limits for each ratio of the test vs. reference formulation were contained well within the established range (80-125%) for bioequivalence. All three prototype formulations were rapidly absorbed on average, with Tₘₐₓ values within 40 minutes post-dose.

### DISCUSSION AND OVERALL CONCLUSIONS

This pilot study compared the rate and extent of ibuprofen absorption from three prototype sodium ibuprofen formulations to the reference standard. All three prototypes were determined to be bioequivalent to the reference standard with respect to AUCL and Cₘₐₓ, and all three prototypes were rapidly absorbed, with times to peak plasma concentration (Tₘₐₓ) within 40 minutes of dosing. Further, times to peak plasma concentration (Tₘₐₓ), times to minimum effective plasma concentration (T_{mec}), and times to plasma concentration of 20 mcg/mL (T₂₀) were faster for the three sodium ibuprofen prototypes compared to the reference standard.

These data are consistent with an earlier PK study comparing the absorption profile of another sodium ibuprofen product to reference standard, ibuprofen lysinate and conventional ibuprofen, which demonstrated that sodium ibuprofen was bioequivalent to the reference standard and ibuprofen lysinate for Cₘₐₓ and AUC with a slightly faster Tₘₐₓ. In addition, this study found that sodium ibuprofen was bioequivalent to conventional ibuprofen for AUC, but was absorbed faster (higher Cₘₐₓ and faster Tₘₐₓ). Since this other formulation of sodium ibuprofen also provided faster onset of analgesia than standard ibuprofen tablets, these data suggest that the sodium ibuprofen tablets tested in the present study may provide an onset of analgesia faster than standard ibuprofen tablets, and at least as fast as the reference standard.

The three sodium ibuprofen prototype formulations and the reference standard evaluated in this pilot study were all well tolerated.

The present invention is not to be limited in scope by the specific embodiments described herein. Indeed, various modifications of the invention in addition to those described herein will become apparent to those skilled in the art from the foregoing description and the accompanying figures. Such modifications are intended to fall within the scope of the appended claims.

It is further to be understood that all values are approximate, and are provided for description.

## Claims

1. A pharmaceutical composition in the form of a tablet or caplet, comprising a core, said core comprising sodium ibuprofen di-hydrate and at least two binders including microcrystalline cellulose and mannitol; said composition comprising at least one coating; wherein said coated core has a sodium content of less than 23 mg/dosage unit.

2. The pharmaceutical composition according to claim 1, wherein the sodium ibuprofen di-hydrate is present in an amount from 50 to 90% by weight, based on the weight of the core of the pharmaceutical composition.

3. The pharmaceutical composition according to claim 1 or claim 2, wherein the at least two pharmaceutically acceptable binders and other excipients are present in an amount from 10 to 50% by weight, based on the weight of the core of the pharmaceutical composition.

4. The pharmaceutical composition according to any one of the preceding claims, having a hardness of greater than 30 N.

5. The pharmaceutical composition according to any one of the preceding claims, having a hardness of greater than 40 N.

6. The pharmaceutical composition according to any one of the preceding claims, having a hardness of greater than 80 N.

7. The pharmaceutical composition according to any one of the preceding claims, wherein the one or more pharmaceutically acceptable coatings is present in an amount from 0.1 to 10% by weight, based on the weight of the core of the pharmaceutical composition.

8. A pharmaceutical composition as claimed in any one of the preceding claims, wherein said composition provides a total daily sodium content for a patient of less than 134 mg/day.

9. A pharmaceutical composition as claimed in any one of the preceding claims, which is in the form of a tablet, wherein said core consists of:
(a) granules consisting of sodium ibuprofen dihydrate, mannitol, and colloidal silicon dioxide in a w/w ratio of 200 : 51.6 : 2.34 respectively; which are compressed with
(b) a mixture of mannitol, microcrystalline cellulose, colloidal silicon dioxide and sodium lauryl sulphate in a w/w ratio of 48.78 : 30.93 : 1.56 : 0.39 respectively wherein components (a) and (b) are present in a w/w ratio of 253.95 : 81.66.

10. A pharmaceutical composition as claimed in claim 9, wherein the granules are produced by roller compaction and milling.

11. A pharmaceutical composition as claimed in claim 9 or claim 10, wherein the cores are coated with a film coat.

12. A pharmaceutical composition as claimed in any one of the preceding claims, wherein the sodium ibuprofen di-hydrate is racemic sodium ibuprofen di-hydrate.

## Patentansprüche

1. Pharmazeutische Zusammensetzung in Form einer Tablette oder Kapsel, umfassend einen Kern, wobei der Kern Natrium-Ibuprofen-Dihydrat und mindestens zwei Bindemittel, einschließlich mikrokristalline Zellulose und Mannitol umfasst; wobei die Zusammensetzung mindestens eine Beschichtung umfasst; wobei der beschichtete Kern ein Natriumgehalt von weniger als 23 mg/Dosierungseinheit aufweist.

2. Pharmazeutische Zusammensetzung nach Anspruch 1, wobei das Natrium-Ibuprofen-Dihydrat in einer Menge von 50 bis 90 Gew.-%, bezogen auf das Gewicht des Kerns der pharmazeutischen Zusammensetzung, vorliegt.

3. Pharmazeutische Zusammensetzung nach Anspruch 1 oder Anspruch 2, wobei die mindestens zwei pharmazeutisch annehmbaren Bindemittel und weitere Hilfsstoffe in einer Menge von 10 bis 50 Gew.-%, bezogen auf das Gewicht des Kerns der pharmazeutischen Zusammensetzung, vorliegen.

4. Pharmazeutische Zusammensetzung nach einem der vorangehenden Ansprüche mit einer Härte von größer als 30 N.

5. Pharmazeutische Zusammensetzung nach einem der vorangehenden Ansprüche mit einer Härte von größer als 40 N.

6. Pharmazeutische Zusammensetzung nach einem der vorangehenden Ansprüche mit einer Härte von größer als 80 N.

7. Pharmazeutische Zusammensetzung nach einem der vorangehenden Ansprüche, wobei die eine oder mehreren pharmazeutisch annehmbare(n) Beschichtung(en) in einer Menge von 0,1 bis 10 Gew.-%, bezogen auf das Gewicht des Kerns der pharmazeutischen Zusammensetzung, vorliegt/vorliegen.

8. Pharmazeutische Zusammensetzung, wie in einem der vorangehenden Ansprüche beansprucht, wobei die Zusammensetzung für einen Patienten eine tägliche Gesamtnatriummenge von weniger als 134 mg/Tag bereitstellt.

9. Pharmazeutische Zusammensetzung, wie in einem der vorangehenden Ansprüche beansprucht, die in der Form einer Tablette ist, wobei der Kern besteht aus:
(a) ein Granulat, bestehend aus Natrium-Ibuprofen-Dihydrat, Mannitol und kolloidalem Siliciumdioxid in einem Gewichtsverhältnis von 200 : 51,6 : 2,34; das komprimiert ist mit
(b) einer Mischung aus Mannitol, mikrokristalliner Zellulose, kolloidalem Siliciumdioxid und Natriumlaurylsulfat in einem Gewichtsverhältnis von 48,78 : 30,93 : 1,56 : 0,39, wobei die Bestandteile (a) und (b) in einem Gewichtsverhältnis von 253,95 : 81,66 vorliegen.

10. Pharmazeutische Zusammensetzung, wie in Anspruch 9 beansprucht, wobei das Granulat durch Walzenverdichtung und Vermahlen hergestellt wird.

11. Pharmazeutische Zusammensetzung, wie in Anspruch 9 oder Anspruch 10 beansprucht, wobei die Kerne mit einer Filmbeschichtung beschichtet sind.

12. Pharmazeutische Zusammensetzung, wie in einem der vorangehenden Ansprüche beansprucht, wobei das Natrium-Ibuprofen-Dihydrat ein racemisches Natrium-Ibuprofen-Dihydrat ist.

## Revendications

1. Composition pharmaceutique sous la forme d'un comprimé ou d'un caplet, comprenant un noyau, ledit noyau comprenant de l'ibuprofène sodique dihydraté et au moins deux liants incluant de la cellulose microcristalline et du mannitol ; ladite composition comprenant au moins un revêtement ; dans laquelle ledit noyau revêtu présente une teneur en sodium inférieure à 23 mg/unité posologique.

2. Composition pharmaceutique selon la revendication 1, dans laquelle l'ibuprofène sodique dihydraté est présent à hauteur de 50 à 90 % en poids, sur la base du poids du noyau de la composition pharmaceutique.

3. Composition pharmaceutique selon la revendication 1 ou la revendication 2, dans laquelle lesdits au moins deux liants pharmaceutiquement acceptables et d'autres excipients sont présents à hauteur de 10 à 50 % en poids, sur la base du poids du noyau de la composition pharmaceutique.

4. Composition pharmaceutique selon l'une quelconque des revendications précédentes, présentant une dureté supérieure à 30 N.

5. Composition pharmaceutique selon l'une quelconque des revendications précédentes, présentant une dureté supérieure à 40 N.

6. Composition pharmaceutique selon l'une quelconque des revendications précédentes, présentant une dureté supérieure à 80 N.

7. Composition pharmaceutique selon l'une quelconque des revendications précédentes, dans laquelle ledit ou lesdits revêtements pharmaceutiquement acceptables sont présents à hauteur de 0,1 à 10 % en poids, sur la base du poids du noyau de la composition pharmaceutique.

8. Composition pharmaceutique telle que revendiquée dans l'une quelconque des revendications précédentes, dans laquelle ladite composition apporte à un patient une quantité totale quotidienne de sodium inférieure à 134 mg/jour.

9. Composition pharmaceutique telle que revendiquée dans l'une quelconque des revendications précédentes, qui se présente sous la forme d'un comprimé, dans laquelle ledit noyau est constitué :
(a) de granulés formés d'ibuprofène sodique dihydraté, de mannitol et de dioxyde de silicium colloïdal selon un rapport pondéral de 200/51,6/2,34 respectivement ; qui sont comprimés avec
(b) un mélange de mannitol, de cellulose microcristalline, de dioxyde de silicium colloïdal et de laurylsulfate de sodium selon un rapport pondéral de 48,78/30,93/1,56/0,39 respectivement
dans laquelle les composants (a) et (b) sont présents selon un rapport pondéral de 253,95/81,66.

10. Composition pharmaceutique telle que revendiquée dans la revendication 9, dans laquelle les granulés sont produits par compactage par rouleau et broyage.

11. Composition pharmaceutique telle que revendiquée dans la revendication 9 ou la revendication 10, dans laquelle les noyaux sont revêtus d'un revêtement pelliculé.

12. Composition pharmaceutique telle que revendiquée dans l'une quelconque des revendications précédentes, dans laquelle l'ibuprofène sodique dihydraté est de l'ibuprofène sodique dihydraté racémique.
